# DEMANDE DE BREVET EUROPEEN

(11) **EP 1 022 555 A2**
(43) Date de publication de la demande: **26.07.2000**
(21) Numéro de dépôt: 00460003.7
(22) Date de dépôt: 14.01.2000
(51) Int. Cl.: G01N 3/56, E02D 1/02

(54) **Dispositifs pour la mesure de l'erodabilité de sediments cohésifs ou de sédiments fins**

(30) Priorité: 21.01.1999 FR 9900889
(71) Demandeur: INSTITUT FRANCAIS DE RECHERCHE POUR L'EXPLOITATION DE LA MER (IFREMER), 92138 Issy-les-Moulineaux Cedex (FR)
(72) Inventeur: Paillard, Michel, IFREMER, Service Mécanique, de Brest-Iroise, 29280 Plouzane (FR); Morel, Jean-Pierre, IFREMER, Laboratoire, de Brest-Iroise, 29280 Plouzane (FR); Le Moign, Tanguy IFREMER, Service Qualification, 29280 Plouzane (FR)
(74) Mandataire: Ballot, Paul

(57) **Abrégé**

L'invention consiste en des moyens permettant des mesures relatives à l'érodabilité effectuées sur des échantillons non remaniés et prenant en compte la rugosité des sédiments.

Ces moyens comprennent une cellule de mesure (1) de forme parallélépipédique dans laquelle est formée une boucle d'écoulement pour générer un écoulement 2D dans une zone de mesure constituée par un canal (31) à la base de ladite boucle.

Selon un autre aspect de l'invention, le fond de la celule (1) peut être constitué par la partie inférieure (5a) formant réceptacle d'un appareil de prélèvement d'échantillon.

## Description

La présente invention concerne la réalisation de mesures relatives à l'érodabilité de sédiments cohésifs ou de sédiments fins.

Par le passé, les mesures d'érodabilité étaient effectuées en laboratoire par remise en suspension portant sur des vases artificielles ou naturelles remaniées, posées au fond d'un canal soumis à un courant ou à des vagues contrôlées. Cependant, de nombreuses observations ont montré une forte variabilité de l'érodabilité d'un sédiment en fonction de multiples paramètres (nature, granulométrie, température, densité, caractéristiques physico-chimiques, rugosité de surface, présence d'organismes, etc.), et finalement en fonction de son histoire (état de consolidation, assèchement, liquéfaction, etc.). Il n'est en fait pas possible de reconstituer le sédiment "naturel" avec toutes ses caractéristiques en laboratoire, ni même de le conserver intact lors d'un transfert en laboratoire depuis une zone de prélèvement. Par conséquent, les mesures en laboratoire ne peuvent rendre compte fidèlement du comportement des sédiments qu'il convient donc d'aborder in situ.

Il existe à ce jour un certain nombre de dispositifs de mesure in-situ de l'érodabilité des sédiments. De façon générale, ces dispositifs sont destinés à fournir une tension de cisaillement critique à partir de laquelle l'érosion du sédiment commence, et/ou un flux d'érosion en fonction du forçage exercé, en général paramétré par la tension de cisaillement sur le fond.

Ces dispositifs connus se présentent sous différentes formes qui peuvent se définir comme suit :
- les canaux ouverts au sein desquels l'écoulement est forcé, mais alimenté par l'eau ambiante pour les systèmes submergés, ou par un réservoir pour les systèmes émergés sur estran;
- les canaux à recirculation qui peuvent être circulaires, présentant une surface de contact avec le sédiment couvrant toute la veine de circulation, utilisables en immersion ou en émersion sur estran; ou bien d'une forme autre, l'interface du sédiment testé étant soumise à un écoulement rectiligne ;
- les cellules à forçage contrôlé, mais sans rapport avec un écoulement naturel, telle celle décrite dans le brevet US n° 4 884 892 au nom de Hydro Data, Inc., où la circulation d'eau est concentrique et complétée par une succion centrale.

La plupart de ces dispositifs sont conçus pour une utilisation exclusive sur estran découvert, ou bien au contraire en immersion sur le fond. Dans tous les cas, ils sont soit trop lourds de mise en oeuvre et/ou trop grands, soit trop restrictifs dans leur usage. D'autre part, la représentativité et l'homogénéité du forçage dans la cellule de mesure constitue une question très délicate et, dans tous les cas, l'effet de la rugosité du fond sédimentaire est ignoré. Ce paramètre est pourtant important, compte tenu des irrégularités naturelles, en particulier d'origine biologique.

En réalité, il existe une demande pour une solution plus globalement satisfaisante à ce problème de mesure d'érodabilité, notamment de la part des scientifiques concernés qui ont conscience de la forte variabilité de l'érodabilité des sédiments superficiels, en particulier en fonction de paramètres biologiques difficilement reproductibles en laboratoire.

C'est en vue de répondre à cette demande qu'a été réalisée l'invention qui, au vu de l'existant, se base sur le fait de prévoir la réalisation de mesures en pseudo in-situ, c'est-à-dire en léger différé sur des sédiments échantillonnés non remaniés, juste après prélèvement, en vue notamment de satisfaire les objectif suivants:
- opérer sur un échantillon non remanié en maintenant au mieux les conditions initiales en termes de rigidité et de rugosité du milieu, tant dans le cas où l'échantillon est issu de l'estran découvert que dans celui où il provient d'un fond immergé;
- bénéficier d'une facilité et d'une rapidité de mise en oeuvre permettant notamment la réalisation de plusieurs essais lors d'une même sortie sur estran ou en mer;
- effectuer un suivi de l'érosion dans des conditions de laboratoire ;
- avoir la possibilité de contrôler de façon optimale la qualité des mesures effectuées ;
- travailler dans des conditions satisfaisantes, avec un minimum de contraintes d'encombrement, de poids, d'énergie, etc.

Partant de ce principe, l'invention se rapporte, d'une part, à un ensemble consistant en l'association d'un dispositif de mesure proprement dit comprenant une cellule de mesure et ses capteurs, et d'un appareil de prélèvement d'échantillon, avec des moyens pour assurer un positionnement précis de l'échantillon prélevé à la base de ladite cellule de mesure, sans altération de celui-ci.

L'invention concerne d'autre part un dispositif de mesure en lui-même, particulièrement adapté pour être intégré dans un tel ensemble, mais qui présente également de par sa nouveauté un intérêt pour une utilisation dans un cadre plus classique, par exemple en laboratoire.

Selon l'invention, ce dispositif de mesure, destiné à la réalisation de mesures relatives à l'érosion d'un sédiment soumis à une tension de cisaillement représentative de l'action d'un courant, et prenant en compte de la rugosité dudit sédiment, telles que les mesures du seuil d'érosion et du flux d'érosion, est caractérisé en ce que ladite cellule de mesure est conçue en vue de la génération d'un écoulement 2D dans la zone de mesure, ladite cellule comprenant une enceinte en forme générale de parallélépipède rectangle, fermée supérieurement par un couvercle comportant un orifice d'admission et un orifice de refoulement, et dans laquelle est définie par un profil intérieur central une boucle de circulation verticale de forme générale en U, la zone de mesure se trouvant sous la base du U et consistant en un canal de hauteur sensiblement constante entre le dessous dudit profil et la surface d'un échantillon contenu dans un fond amovible de ladite cellule.

Selon d'autres caractéristiques relatives à des formes de réalisation préférées de ladite cellule :
- ladite zone de mesure couvre une surface d'échantillon de l'ordre de 100 cm²;
- l'écartement entre le dessous dudit profil et le fond de la cellule est choisi de telle manière que la hauteur dudit canal soit en moyenne de l'ordre de 20 mm ;
- le fond de la cellule est réglable en hauteur par rapport au corps de celle-ci afin de régler avec précision la hauteur dudit canal;
- le fond de la cellule est réglable en assiette par rapport au corps de la cellule, afin de permettre d'uniformiser au mieux la hauteur dudit canal sur toute l'étendue de la zone de mesure;
- les parois de la cellule sont au moins pour partie transparentes pour permettre une observation visuelle de l'érosion à l'intérieur
- deux lignes de détection de pression sont prévues dans ladite zone de mesure, près de ses extrémités amont et aval, pour mesurer la différence de pression entre ces deux lignes, la tension de cisaillement sur le fond étant déduite de cette différence de pression et du débit dans la cellule.

De même, des caractéristiques préférentielles se rapportant à un ensemble selon l'invention regroupant un dispositif de mesure et un appareil de prélèvement sont les suivantes:
- la partie inférieure de l'appareil de prélèvement destinée à recevoir un échantillon est prévue amovible et complémentaire de la base du corps de la cellule de mesure pour en constituer le fond;
- ledit ensemble comprend en outre un carottier destiné au prélèvement d'un échantillon primaire immergé, pour permettre le prélèvement dans ce dernier, dans des conditions optimales, d'un échantillon final avec ledit appareil de prélèvement; le carottier comporte un couvercle afin d'éviter des perturbations au-dessus de l'échantillon primaire qui seraient susceptibles de l'altérer lors de la remontée.

Ces caractéristiques et avantages de l'invention, ainsi que d'autres, apparaîtront plus clairement à la lecture de la description suivante, faite en relation avec les dessins joints, dans lesquels:
la Fig. 1 est une vue schématique en perspective d'un dispositif de mesure selon l'invention, dans une forme expérimentale d'étalonnage initial;
la Fig. 2 est une vue schématique en coupe verticale longitudinale d'une forme de réalisation de cellule de mesure selon l'invention;
la Fig. 3 est une vue de bout schématique de la même cellule de mesure;
la Fig. 4 est une vue schématique de face d'un appareil de prélèvement d'échantillon faisant partie d'un ensemble selon l'invention pour la réalisation de mesures sur échantillon non remanié;
la Fig. 5 est une vue de bout du même appareil de prélèvement, vu de la droite en considérant la Fig. 2;
la Fig. 6 est une vue schématique en coupe représentant une cellule de mesure de même type que celle des Figs. 2 et 3, faisant partie d'un ensemble selon l'invention pour la réalisation de mesures sur échantillon non remanié, et comportant en tant que fond, la partie inférieure de l'appareil de prélèvement des Figs. 4 et 5; et
la Fig. 7 est une vue schématique sous forme de circuit d'un ensemble de mesure comprenant la cellule de mesure de la Fig. 6.

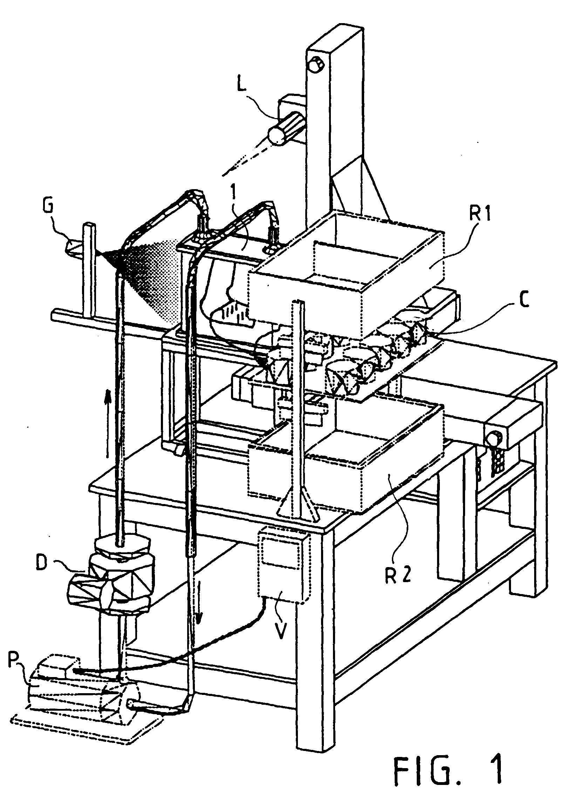

Le dispositif représenté à la Fig. 1 est un ensemble expérimental ayant été constitué pour effectuer des essais, mais également à des fins de validation de la conception de la cellule de mesure 1 et de son aménagement intérieur, et d'étalonnage de celle-ci. Il comprend donc des éléments qui n'auront plus lieu d'être dans sa forme finale, tels la sonde laser L avec son mécanisme de déplacement en X/Y/Z, et le générateur de tranche laser G ayant servi tous deux à des fins d'étalonnage et de validation du modèle théorique.

Outre ces éléments, le dispositif de la Fig. 1 comprend un circuit hydraulique établi entre l'entrée et la sortie de la cellule 1 et présentant en série, dans le sens du flux, une pompe P avec un variateur V et un débitmètre D, avantageusement de type électromagnétique. N'a pas été représenté sur ce dessins un néphélomètre principalement nécessaire pour la mesure du flux d'érosion. Peuvent également être prévus d'autres éléments tels que des moyens de recyclage et des moyens de débullage de l'eau.

Le dispositif expérimental comporte en outre des capteurs de pression C servant à des mesures dans la cellule 1, un bac supérieur R1 qui est un réservoir de purge et de référence, et un réservoir R2 qui est un réservoir de récupération.

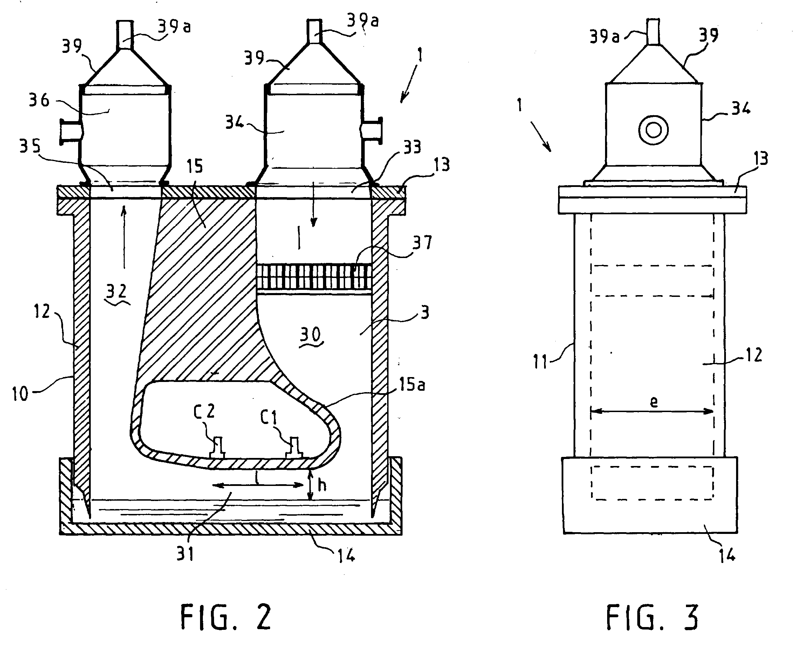

Les Figs. 2 et 3 représentent la cellule 1 dans une forme de réalisation ayant donné des résultats satisfaisants. Elle a la forme générale d'un parallélépipède rectangle, constitué d'un corps 10 formé de deux parois latérales 11 et de deux parois d'extrémité 12, lequel corps 10 est fermé de façon étanche, supérieurement par un couvercle 13, et intérieurement par un fond amovible 14. Selon une caractéristique importante de l'invention, les parois de la cellule 1 sont au moins en partie transparentes pour permettre une observation visuelle de l'érosion et l'étalonnage préliminaire par vélocimétrie laser.

Dans le volume intérieur de la cellule 1, un élément 15 que l'on dénommera profil dans la suite a un contour extérieur formé par une paroi perpendiculaire aux parois latérales 11, jointe continûment à chacune de celle-ci, et jointe également par ses extrémités au couvercle 13. Le profil 15 a pour vocation de former à l'intérieur de la cellule, une boucle de circulation verticale en U 3. La boucle 3 est prolongée par un orifice d'admission 33 dans le couvercle 13, en amont duquel est avantageusement prévu une colonne d'entrée 34 ; et en son autre extrémité par un orifice de sortie 35 dans le couvercle 13, ouvert dans une colonne de sortie 36. A partir de leur base et comme le montrent les Fig. 2 et 3, les colonnes 34 et 36 ont une section évoluant rapidement de la forme rectangulaire ou carrée à la forme circulaire. Elle sont obturées supérieurement par un bouchon conique 39 en forme d'entonnoir inversé, dont la queue cylindrique 39a constitue une purge éventuellement pourvue d'une vanne d'ouverture/fermeture, destinée à favoriser le débullage.

Le fond 14 de la cellule 1 est conçu pour recevoir un échantillon à mesurer, lequel constituera donc le fond pour la boucle de circulation 3. Le fond 14 est ici représenté sous la forme d'un bac enveloppant par ses bords la base du corps 10, mais il pourrait naturellement être différent. De préférence, des moyens sont prévus pour régler le positionnement du fond 14 par rapport au corps 1, dans le sens de la hauteur et aussi relativement à son assiette.

La boucle de circulation 3 est conçue de manière à générer un écoulement 2D dans une zone de mesure 31 sous le profil 15. La zone de mesure 31 est un canal de section rectangulaire constante délimitée par l'échantillon A tester, les parois latérales 11 et le dessous du profil 15. Le profil 15 est d'autre part conçu de manière à obtenir entre l'amont et l'aval de la zone de mesure une tension de cisaillement quasi-uniforme et maximale sur le fond, afin que l'érosion soit générée dans ladite zone de mesure. En amont de la zone de mesure 31, le profil 15 est arrondi pour former un bulbe 15a, de telle manière que la branche verticale amont 30 de la boucle 3 forme un premier convergent au niveau de la partie supérieure du bulbe 15a, et un second convergent au niveau de sa partie inférieure, à l'entrée du canal de mesure 31, de façon à obtenir un profil de vitesse quasi-uniforme à l'entrée de ladite zone de mesure.

En aval, le canal 31 se prolonge de façon divergente de par une inclinaison de la paroi du profil 15, laquelle forme ensuite un arrondi et remonte linéairement jusqu'au couvercle 13, selon une inclinaison de divergence pour la branche verticale aval 32 de la boucle 3. A noter que la colonne d'admission 34 est conçue notamment pour éliminer les bulles dans l'eau entrant dans la boucle 3.

Dans la partie supérieure de la branche verticale amont 30 de la boucle 3, est avantageusement prévue une grille 37 de tranquillisation de l'écoulement, celle-ci comprenant avantageusement une structure en nid d'abeille.

Dans la zone de mesure 31, sont prévues respectivement près de son extrémité amont et de son extrémité aval, deux lignes de relevé de pression C1 et C2.

Les valeurs dimensionnelles suivantes relatives à la cellule 1 sont données à titre d'exemple non limitatif, pour illustrer notamment l'importante différence de gabarit avec les dispositifs existants, les dimensions de longueur correspondant à la direction longitudinale du canal de mesure 31:
- longueur interne de la cellule : 255 mm
- largeur interne de la cellule : 100 mm
- hauteur jusqu'au couvercle 13 : 300 mm
- longueur du resserrement dans la branche 30 au niveau du sommet du bulbe 15a : 50 mm
- rayon de courbure du bulbe 15a : 30 mm
- section haute de la branche 30 : 80 mm X 100 mm
- section haute de la branche 32 : 50 mm X 100 mm
- angle de divergence du profil 15 en aval de la zone de mesure : 8°
- angle de divergence du profil 15 le long de la branche 32 : 8°

Le canal de mesure 31 a donc une largeur e de 100 mm, et sa longueur 1 entre C1 et C2 a ici été choisie égale à 70 mm. La hauteur h du canal de mesure 31 (écartement entre l'échantillon à tester et le dessous du profil 15) peut être fixée à 20 mm, environ, de préférence avec la possibilité de la faire varier légèrement en prenant soin de la mesurer.

Le dispositif de mesure selon l'invention avec une cellule de mesure telle que décrite ci-dessus permet donc une estimation de la tension de cisaillement sur un échantillon en tenant compte de la rugosité du sédiment. Pour déterminer cette contrainte, des mesures de débit et de différence moyenne entre les pressions aux extrémités de la zone de mesure sont effectuées et la procédure suivante est adoptée:
- étalonnage préalable de l'écoulement dans la cellule via une mesure de vélocimétrie laser effectuée avec des moyens tels que représentés à la Fig. 1; validation des paramètres du modèle théorique de l'écoulement dans la zone de mesure (influence de l'écoulement le long des coins de la zone de mesure); établissement de la corrélation définissant la tension de cisaillement en fonction du débit et de la mesure des pressions aux extrémités de la zone de mesure, par étude paramétrique du modèle théorique en fonction du débit et de la rugosité du sédiment, en utilisant un jeu de semelles à rugosité uniforme et connue à disposer en lieu et place du sédiment;
- prélèvement, mise en place de l'échantillon sous le corps de la cellule et réglage de la hauteur h du canal de mesure;
- mise en route de l'écoulement et mesure par palier de vitesse de pompe;
- à chaque palier, mesure du débit et de la différence de pression; calcul de la tension de cisaillement à l'aide de la corrélation précitée.

Le seuil d'érosion peut être détecté au moyen d'un néphélomètre servant également à mesurer le flux d'érosion, mais selon l'invention, le seuil d'érosion peut également être détecté avantageusement par visualisation directe ou au moyen d'une caméra dans la zone de mesure, à travers les parois transparentes.

Le remplissage en eau de la cellule 1 préalable à une opération de mesure s'effectue à l'aval de la partie active, progressivement, et de préférence par tranches successives, de façon à éviter à la fois une érosion ponctuelle préalable de l'interface sédimentaire ainsi que la génération de bulles d'air dans le circuit pouvant perturber la mesure ultérieure de pression différentielle. En pratique, le remplissage peut être effectué en trois tranches, les première et seconde via des piquages respectivement bas et haut dans la paroi de la cellule 1 le long de la branche aval 32, et la troisième via la purge 39a dans le haut des colonnes 34 et 36.

La vidange de la cellule après une opération de mesure s'effectue également de la même façon par tranches successives si l'on veut conserver après démontage du réceptacle d'échantillon 14 la trace de la figure d'érosion résultant de ladite opération. L'examen de la trace peut permettre de valider l'opération et d'apporter des éléments complémentaires d' interprétation.

La cellule décrite précédemment est particulièrement adaptée pour s'intégrer dans un ensemble qui, selon un autre aspect de l'invention déjà explicité ci-avant, consiste en l'association d'un dispositif de mesure et d'un appareil de prélèvement permettant d'opérer sur des échantillons non remaniés dans des conditions optimales. Un point du processus de mise en oeuvre est en effet particulièrement délicat, s'agissant de la mise en place de la cellule de mesure sur un échantillon contenu dans l'appareil de prélèvement.

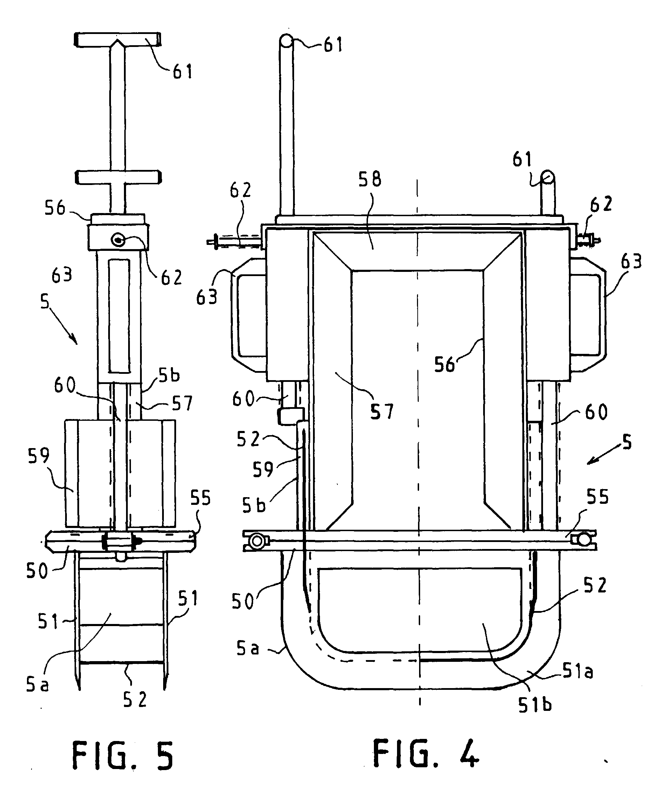

Une solution apportée par l'invention est illustrée schématiquement aux Figs. 4 à 6, selon laquelle l'appareil de prélèvement 5 a été conçu de telle façon que sa partie inférieure 5a, prévue étanche, soit amovible et puisse venir se positionner sous le corps de la cellule 1 pour en constituer le fond.

Plus précisément, l'appareil de prélèvement 5 comprend outre sa partie inférieure 5a de réception d'un échantillon, une partie supérieure de manoeuvre 5b.

La partie inférieure 5a constituant le réceptacle d'échantillon comporte une ceinture supérieure 50 à laquelle sont jointes deux parois ou joues 51 dirigées parallèlement vers le bas. Les joues 51 coopèrent avec deux volets 52 disposés symétriquement de chaque côté pour former un volume intérieur de réception d'échantillon. Les volets 52 sont des lames métalliques flexibles engagées sur leurs deux côtés dans des rainures périphériques ménagées dans la face interne des joues 51. Les volets 52 ont une aptitude de coulissement entre une position d'ouverture et une position de fermeture illustrées respectivement dans la partie de gauche et la partie de droite de la Fig. 4. Par ailleurs, les joues 51 présentent des fenêtres transparentes 51b, avec de préférence des repères de niveau non représentés.

En position d'ouverture des volets 52, ceux-ci, avec les joues 51 dont le bord extérieur 51a est biseauté à cet effet, forment un ensemble propre à être enfoncé dans un sédiment dans les conditions requises pour une prise d'échantillon satisfaisante, notamment du point de vue de la qualité de découpe du bord extérieur en surface de l'échantillon. En position fermée des volets 52, ceux-ci forment avec les joues 51 un réceptacle étanche pour cet échantillon.

La partie supérieure 5b de l'appareil 5 est la partie de manoeuvre. Elle comporte une ceinture 55 complémentaire de la ceinture 50 de la partie inférieure 5a, laquelle ceinture 55 porte un châssis 56 en forme de portique constitué de deux montants 57 réunis par une traverse 58. Les montants 57 portent extérieurement en partie basse des glissières 59 recevant les volets 52 lors de leur ouverture. Les montants 57 portent en outre des bras 60 d'entraînement des volets 52, qui se prolongent en des poignées d'actionnement 61, pourvues de moyens de verrouillage en position de fermeture 62. Les poignées 63 sont fixes sur le châssis 56 et servent à la préhension de l'appareil.

Conformément à l'invention, la partie basse ou réceptacle d'échantillon 5a est donc libérable de la partie supérieure 5b, par séparation des deux ceintures complémentaires 50 et 55, entre lesquelles sont prévus des moyens d'assemblage à libération rapide. Sont en outre prévus des moyens pour assurer que d'une part, lorsqu'ils sont en position de fermeture, les volets 52 soient libérés de leurs bras d'actionnement 60 et verrouillés dans la ceinture 50, et que d'autre part, lors de l'assemblage des ceintures 50 et 51, les bras 60 soient réaccouplés avec les volets 52.

Pour réaliser un prélèvement, l'appareil 5 ouvert, tenu par les poignées 63, est enfoncé dans le sédiment jusqu'à atteindre le niveau et le positionnement souhaités de la surface de l'échantillon dans le réceptacle 5a, par repérage à travers les fenêtres 51b des joues 51. Les volets 52 sont ensuite refermés par actionnement et verrouillage des poignées 61. L'appareil étant alors relevé, le réceptacle 5a chargé de son échantillon peut être séparé de la partie supérieure 5b. On notera qu'il est possible d'associer cette dernière à un jeu de plusieurs réceptacles 5a, pour permettre de multiplier les prélèvements lors d'une même sortie. Malgré la surface limitée des échantillons, on peut ainsi obtenir des mesures représentatives d'une surface beaucoup plus importante, en bénéficiant au surplus d'indications intéressantes sur leur variabilité. A noter également que les réceptacles 5a peuvent être pourvus d'un couvercle étanche pour permettre le transport de l'échantillon sans remaniement.

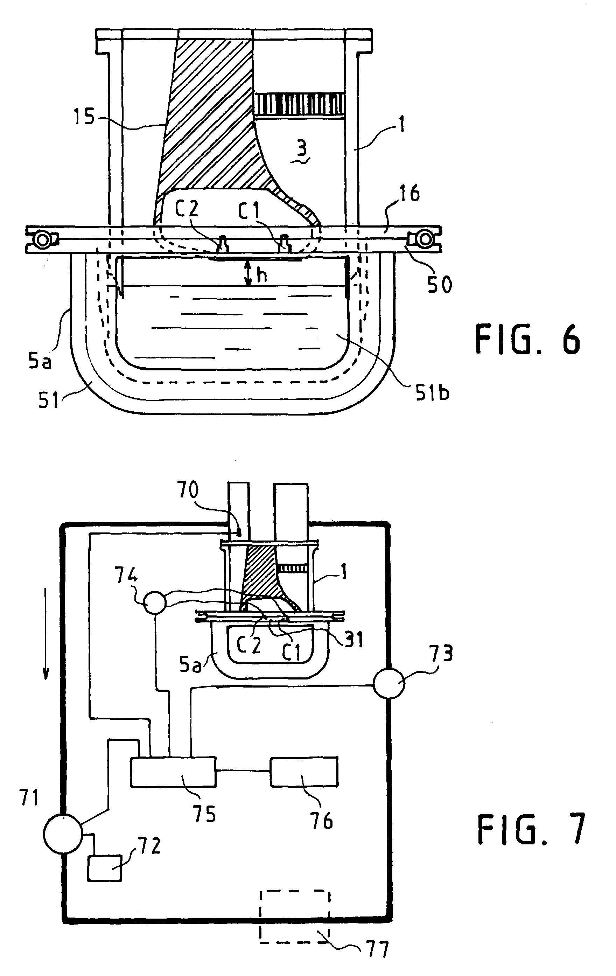

La Fig.6 illustre schématiquement un exemple de ce que peut être la complémentarité entre une cellule 1 et l'appareil de prélèvement 5 décrits plus haut, associés au sein d'un ensemble de mesure selon l'invention. Le réceptacle 5, chargé d'un échantillon admet intimement dans son volume intérieur la base de la cellule 1 dont le bord inférieur des parois peut être biseauté pour rentrer légèrement dans l'échantillon. A un niveau judicieusement déterminé de sa hauteur, la cellule 1 comporte une ceinture périphérique 16 sous laquelle vient se placer la ceinture 50 du réceptacle 5b. De préférence, des moyens étanches de réglage et de mesure, non représentés, sont prévus entre les ceintures 16 et 50 pour ajuster et mesurer au mieux la hauteur h du canal de mesure et sa constance sur toute l'étendue de la zone de mesure. A noter qu'ici, l'ensemble de la zone de mesure est au-dessous de la ceinture 50 du réceptacle 5b, et donc entièrement visible à travers la fenêtre 51b des joues 51.

Le dispositif de mesure selon l'invention schématisé à la Fig. 7 est un circuit en boucle fermée comprenant d'amont en aval entre les orifices de refoulement et d'admission de la cellule 1 : un néphélomètre 70 placé de préférence immédiatement à la suite et dans l'axe de l'orifice de refoulement 35, une pompe 71 avec un variateur 72, et un débitmètre électromagnétique 73. Un capteur de pression 74 mesure la pression différentielle entre les lignes C1 et C2 aux extrémités de la zone de mesure dans la cellule 1. Tous ces composants sont reliés à une centrale d'acquisition de données 75, reliée elle-même, avec le variateur 72, à un micro-ordinateur 76. En variante d'une boucle fermée, le circuit peut être ouvert entre un réservoir amont et une sortie d'écoulement aval.

D'autre part, à la place du néphélomètre 70 ou en redondance avec lui, il peut être prévu une enceinte d'homogénéisation avec néphélémétre à l'intérieur pour la mesure de la variation des flux d'érosion, placée par exemple entre la pompe 71 et le débitmètre 73, comme représenté en traits interrompus et repéré en 77.

Un tel dispositif de mesure peut être installé dans un véhicule, avec les réserves en énergie et en fluide nécessaires pour les opérations envisagées, en vue de mesures à effectuer sur échantillons issus de l'estran qui peuvent ainsi être traités très rapidement après leur prélèvement ; ou bien à bord d'un bateau, pour des mesures relatives à des sédiments immergés. Dans ce second cas, un ensemble de mesure selon l'invention est complété par un carottier destiné au prélèvement d'échantillons primaires de grande dimension dans chacun desquels il peut être extrait ensuite dans des conditions optimales un ou plusieurs échantillons finaux au moyen de l'appareil de prélèvement. Le carottier est bien entendu étanche et, de plus, pourvu d'un couvercle afin d'éviter les turbulences au-dessus de l'échantillon lors de la remontée. L'appareil de prélèvement étant lui-même étanche, il est possible de n'interrompre à aucun moment la condition d'immersion des échantillons.

## Revendications

1. Dispositif destiné à la réalisation de mesures relatives à l'érosion d'un sédiment soumis à une tension de cisaillement représentative de l'action d'un courant et prenant en compte la rugosité dudit sédiment, telle que les mesures du seuil d'érosion et du flux d'érosion, caractérisé en ce qu'il comprend une cellule de mesure (1) conçue en vue de la génération d'un écoulement 2D dans la zone de mesure, ladite cellule (1) comprenant une enceinte en forme générale de parallélépipède rectangle, fermée supérieurement par un couvercle (13) comportant un orifice d'admission (33) et un orifice de refoulement (35), et dans laquelle est définie par un profil intérieur central (15) une boucle de circulation verticale de tonne générale en U (3), la zone de mesure se trouvant sous la base du U et consistant en un canal (31) de hauteur sensiblement constante entre le dessous dudit profil (15) et la surface d'un échantillon contenu dans un fond amovible (14) de ladite cellule (1).

2. Dispositif selon la revendication 1, caractérisé en ce que dans la cellule (1), ladite zone de mesure (31) couvre une surface d'échantillon de l'ordre de 100 cm².

3. Dispositif selon la revendication 1 ou 2, caractérisé en ce que dans la cellule (1), l'écartement entre le dessous dudit profil (15) et le fond (14) est choisi de telle manière que la hauteur dudit canal (31) soit en moyenne de l'ordre de 20 mm.

4. Dispositif selon l'une des revendications 1 à 3, caractérisé en ce que le fond (14) de la cellule (1) est réglable en hauteur par rapport au corps de celle-ci afin de pouvoir faire varier légèrement la hauteur dudit canal (31) de part et d'autre d'une valeur nominale.

5. Dispositif selon l'une des revendications 1 à 4, caractérisé en ce que le fond (14) de la cellule (1) est réglable en assiette par rapport au corps (10) de celle-ci, afin de permettre d'uniformiser au mieux la hauteur dudit canal (31) sur toute l'étendue de la zone de mesure.

6. Dispositif selon l'une des revendications 1 à 5, caractérisé en ce que les parois de la cellule (1) sont au moins pour partie transparentes pour permettre une observation visuelle de l'érosion à l'intérieur.

7. Dispositif selon l'une des revendications 1 à 6, caractérisé en ce que deux lignes de détection de pression (C1, C2) sont prévues dans ladite zone de mesure, près de ses extrémités amont et aval, pour mesurer la différence de pression entre ces deux lignes, la tension de cisaillement sur le fond étant déduite de cette différence de pression et du débit dans la cellule (1).

8. Ensemble destiné à la réalisation de mesures relatives à l'érodabilité de sédiments et conçu pour traiter des échantillons non remaniés, caractérisé en ce qu'il consiste en l'association d'un dispositif proprement dit comprenant une cellule de mesure et ses capteurs, et d'un appareil de prélèvement, avec des moyens pour assurer le positionnement requis des échantillons prélevés à la base de ladite cellule de mesure.

9. Ensemble selon la revendication 8, caractérisé en ce que la partie inférieure dudit appareil de prélèvement destinée à recevoir un échantillon est prévue amovible et complémentaire de la base du corps de la cellule de mesure pour en constituer le fond.

10. Ensemble selon la revendication 8 ou 9, caractérisé en ce qu'il comprend en outre un carottier destiné au prélèvement d'un échantillon primaire immergé, pour permettre le prélèvement dans ce dernier, dans des conditions optimales, d'un échantillon final avec ledit appareil de prélèvement.

11. Ensemble selon l'une des revendications 8 à 10, caractérisé en ce qu'il comprend un dispositif de mesure selon l'une des revendications 1 à 6.
